# EUROPÄISCHE PATENTANMELDUNG

(11) **EP 2 100 638 A1**
(43) Veröffentlichungstag der Anmeldung: **16.09.2009**
(21) Anmeldenummer: 09003442.2
(22) Anmeldetag: 10.03.2009
(51) Int. Cl.: A61M 25/01, A61M 25/06, A61M 25/09

(54) **Katheterbausatz mit einem Führungsdraht**

(30) Priorität: 12.03.2008 DE 102008013884
(71) Anmelder: Osypka, Peter, 79618 Rheinfelden-Herten (DE)
(72) Erfinder: Osypka, Peter, 79618 Rheinfelden-Herten (DE)
(74) Vertreter: Maucher, Wolfgang

(57) **Zusammenfassung**

Ein Katheterbausatz mit einem Führungsdraht (1) und einem über diesen Führungsdraht (1) zu einem Behandlungsort verschiebbaren Behandlungskatheter (3) weist außerdem noch einen an seinem distalen Ende (6) lenkbaren Führungskatheter (7) auf, der ein Innenlumen hat, um den Führungsdraht (1) in sich aufnehmen zu können. Die zunächst als eine Einheit zusammenwirkende Kombination des lenkbaren Führungskatheters (7) und des Führungsdrahtes (1) ermöglicht die Verlegung dieses Führungsdrahtes (1) auch an schwer zugängliche Stellen. Nach dem Zurückziehen des Führungskatheters (7) kann dann über den verlegten Führungsdraht (1) der Behandlungskatheter (3) in Gebrauchsstellung gebracht werden (Fig. 3).

## Beschreibung

Die Erfindung betrifft einen Katheterbausatz mit einem Führungsdraht und mit einem auf diesem Führungsdraht zu seinem Bestimmungsort verschiebbaren Behandlungskatheter, der einen inneren Kanal oder ein Innenlumen aufweist, womit er in Gebrauchsstellung den Führungsdraht umschließt.

Derartige Katheterbausätze oder Katheteranordnungen sind in unterschiedlicher Form und vielfältig bekannt. Der Führungsdraht dient bei einem solchen Katheterbausatz dazu, zunächst verlegt zu werden, damit dem Operateur das Verschieben des Behandlungskatheters an die entsprechende Stelle erleichtert wird, weil der Behandlungskatheter nach dem Verlegen des Führungsdrahtes nur noch auf diesem verschoben werden muss und den Krümmungen und Richtungsänderungen beispielsweise im Inneren eines Blutgefäßes aufgrund der entsprechenden Anordnung des Führungsdrahtes leicht folgen kann. Der relativ dünne Führungsdraht lässt sich dabei einfacher als ein in der Regel einen größeren Querschnitt aufweisende Behandlungskatheter verlegen, weshalb zu sehr vielen Katheterbausätzen ein solcher Führungsdraht gehört.

Es gibt jedoch im menschlichen Körper und insbesondere im Herzen, beispielsweise am Ausflusspunkt des Blutes aus den Koronarvenen, dem sogenannten Koronar-Sinus, Stellen, an die das Hinführen eines Führungsdrahtes praktisch nicht möglich ist, weil diese Stellen für einen gängigen Führungsdraht praktisch nicht zugänglich sind.

Es besteht deshalb die Aufgabe, eine Katheteranordnung oder einen Katheterbausatz der eingangs genannten Art zu schaffen, womit es möglich ist, einen Behandlungskatheter und insbesondere dessen distales Ende auf einfache Weise an einer schlecht oder schwer zugänglichen Stelle im menschlichen Körper, beispielsweise am Koronar-Sinus, zu platzieren.

Zur Lösung dieser Aufgabe ist vorgesehen, dass zu dem eingangs genannten Katheterbausatz zusätzlich zu dessen Teilen ein an seinem distalen Ende lenkbarer Führungskatheter gehört, der einen durchgängigen, an seinem lenkbaren distalen Ende ins Freie mündenden Kanal oder ein Innenlumen aufweist, in welchem der Führungsdraht zumindest bei seiner Verlegung angeordnet und in seine Gebrauchslage verschiebbar ist.

Es wird also eine Katheteranordnung vorgesehen, bei welcher ein zusätzlicher Führungskatheter dazu benutzt werden kann, die im menschlichen Körper nur schwer zugängliche Stelle aufgrund der Lenkbarkeit zu erreichen, so dass der in diesen Führungskatheter passende und in ihm zumindest zeitweise befindliche Führungsdraht bei oder nach dem Verlegen des Führungskatheters ganz leicht vorgeschoben und in Gebrauchslage gebracht werden kann. Mit anderen Worten kann mit Hilfe des zusätzlich zu dem Katheterbausatz gehörenden Führungskatheter sehr schnell eine nur schlecht oder schwierig zugängliche Stelle im menschlichen Körper erreicht werden, so dass mit Hilfe dieses lenkbaren Katheters aufgrund des in ihm befindlichen Kanals ein Führungsdraht verlegt werden kann, der nun in diesem Führungskatheter sehr leicht und schnell die schwer zugängliche Stelle erreicht, wonach dann - nach dem Zurückziehen des lenkbaren Führungskatheters - der Behandlungskatheter in an sich bekannter Weise mit dem Führungsdraht zusammenwirken, also über den Führungsdraht eingeführt oder implantiert und/oder platziert werden kann. Dabei kann der Behandlungskatheter beispielsweise auch eine Elektrode mit entsprechendem Innenkanal oder Innenlumen sein.

Besonders zweckmäßig ist es dabei, wenn der Führungskatheter relativ zu dem in Gebrauchslage befindlichen Führungsdraht zurückziehbar und/oder entfernbar ist, um in der schon erwähnten Weise den Platz für das Einführen des Behandlungskatheters frei zu geben.

Der Kanal oder das Innenlumen des lenkbaren Führungskatheters kann flüssigkeitsdicht sein. Dadurch ist es möglich, den Führungskatheter auch zunächst noch zum Applizieren einer Flüssigkeit, beispielsweise eines Medikaments zu nutzen, so dass dieser zu dem Katheterbausatz gehörende Führungskatheter eine weitere Funktion erhält.

Eine weitere Ausgestaltung des erfindungsgemäßen Katheterbausatzes kann darin bestehen, dass an dem Führungskatheter am oder nahe dem distalen Ende ein aufweitbarer Ballon angeordnet ist. Dieser erlaubt es, den Koronar-Sinus, also den Blutaustritt einer Koronarvene zumindest kurzzeitig zu verschließen, um während der dadurch hervorgehobenen kurzzeitigen Unterbrechung des Blutstroms zum Beispiel mit Hilfe eines Kontrastmittels den anatomischen Bereich im oder auch hinter dem Koronar-Sinus überprüfen und untersuchen zu können, damit das anschließende Platzieren des Führungsdrahtes und danach das Platzieren des Behandlungskatheters oder der Elektrode durch den Koronar-Sinus hindurch erleichtert wird.

Dabei kann im Inneren des Führungskatheters ein zu dem Ballon führender eigener Kanal, Schlauch, Röhrchen oder Leitung für das zum Aufweiten des Ballons dienende Medium vorgesehen sein.

Der lenkbare Führungskatheter kann wenigstens ein exzentrisch zu seiner Längsmitte angeordnetes Zugelement aufweisen, das zu seinem auslenkbaren biegsamen distalen Ende verläuft und durch eine Zug- und/oder Druckkraft zur Auslenkung des distalen Endes des Führungskatheters beaufschlagbar ist. Vor allem dann, wenn das Zugelement so geführt ist, dass auch eine Schubkraft aufgebracht werden kann, kann der Führungskatheter mit seinem distalen Ende nach entgegengesetzten Seiten ausgelenkt werden, was die Lenkbarkeit bei gleichzeitig möglichst geringem Innenquerschnitt erleichtert, da für die Auslenkung nach entgegengesetzten Richtungen dann kein zweites Zugelement benötigt wird. Praktisch kann der lenkbare Führungskatheter insbesondere hinsichtlich der Lenkbarkeit etwa gemäß DE 103 37 580 A1 gestaltet und ausgebildet sein.

Günstig ist es dabei, wenn das Zugelement in einem Führungselement oder Röhrchen verläuft und von diesem sein Ausknicken bei Schubbelastung verhindernd eng umschlossen ist.

Vor allem bei Kombination einzelner oder mehrerer der vorbeschriebenen Merkmale und Maßnahmen ergibt sich eine Katheteranordnung oder ein Katheterbausatz, mit welchem auch schwer zugängliche Bereiche im Inneren des menschlichen Körpers und insbesondere im Inneren des Herzens dadurch gut erreicht werden können, dass für die Verlegung des Führungsdrahtes der zu dem Bausatz gehörende Führungskatheter aufgrund seiner Lenkbarkeit zeitweilig implantiert werden kann, wobei der Führungsdraht gleichzeitig oder danach problemlos durch diesen Führungskatheter in seine Gebrauchslage gebracht werden kann. Anschließend kann dann nach dem Entfernen des Führungskatheters der Behandlungskatheter oder eine zur Behandlung dienende Elektrode in konventioneller Weise über den Führungsdraht implantiert werden.

Nachfolgend wird ein Ausführungsbeispiel der Erfindung anhand der Zeichnung näher beschrieben. Es zeigt in zum Teil schematisierter Darstellung:
- Fig. 1: eine Seitenansicht eines zu dem erfindungsgemäßen Katheterbausatz gehörenden Führungskatheters, der an seinem distalen, auslenkbaren Ende auch einen Ballon trägt,
- Fig. 2: eine der Fig. 1 entsprechende Darstellung, wobei an einem Eintritt in das Innenlumen des Führungskatheters eine Injektionsvorrichtung angeschlossen ist, mit welcher ein Medikament durch den Führungskatheter einführbar ist, wobei gleichzeitig die Auslenkbarkeit des lenkbaren distalen Endes des Führungskatheters nach entgegengesetzten Seiten gezeigt ist,
- Fig. 3: eine den Fig. 1 und 2 entsprechende Darstellung, in welcher an einem weiteren Zutritt zu dem Innenlumen des Führungskatheters ein Führungsdraht eingeschoben ist, der am distalen Ende des Führungskatheters vorsteht,
- Fig. 4: den Führungskatheter mit Führungsdraht gemäß Fig. 3 nach dem Einführen in ein Herz und dabei in den Ko- ronar-Sinus,
- Fig. 5: eine der Fig. 4 entsprechende Darstellung, bei wel-cher der Führungskatheter gegenüber dem zunächst mit seiner Hilfe verlegten Führungsdraht bereits teilweise zurückgezogen ist, sowie
- Fig. 6: eine Darstellung gemäß Fig. 4 und 5, in welcher gezeigt ist, dass nach dem endgültigen Zurückziehen des Führungskatheters über den Führungsdraht ein zu dem Katheterbausatz gehörender Behandlungskatheter oder eine Elektrode mit entsprechenden Verankerungen einführbar ist, wobei die Behand-lungselektrode erst teilweise eingeführt und noch bis zum Ende des Führungsdrahtes weiter verschieb-bar ist.

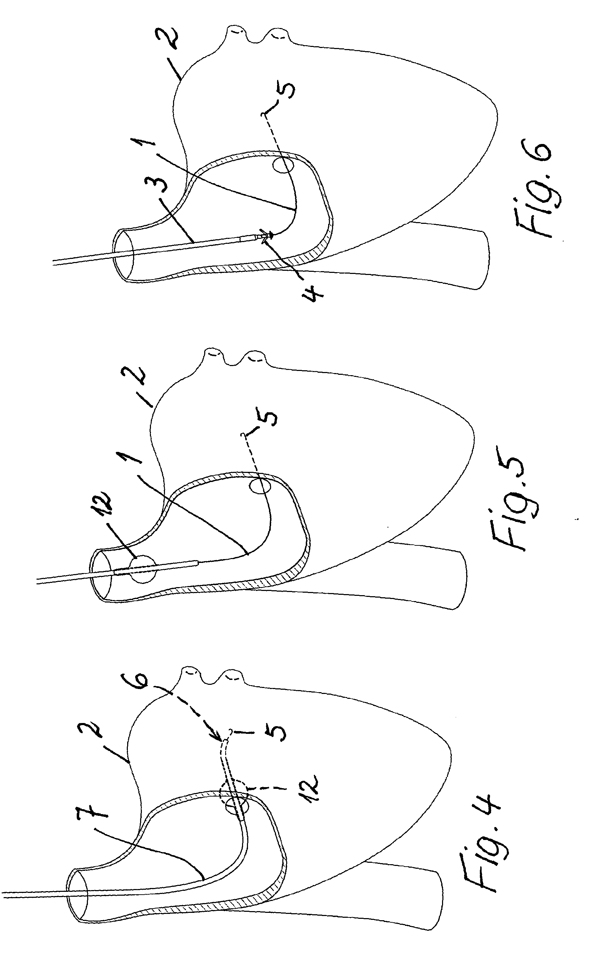

Ein in keiner der Figuren komplett dargestellter Katheterbausatz weist als wesentliche Bestandteile einen Führungsdraht 1 und einen gemäß Fig. 6 auf diesem Führungsdraht 1 zu seinem Bestimmungsort in einem Herzen 2 verschiebbaren Behandlungskatheter 3 auf, der dazu in bekannter Weise einen inneren Kanal oder ein Innenlumen aufweist, womit er in der Gebrauchsstellung gemäß Fig. 6 den Führungsdraht 1 umschließt. Man erkennt an der Elektrode beziehungsweise an dem Behandlungskatheter 3 in Fig. 6 einen Anker 4, womit der Behandlungskatheter 3 nach der endgültigen Implantation, also nach der endgültigen Verschiebung bis zum Ende 5 des Führungsdrahtes 4 festgelegt werden kann.

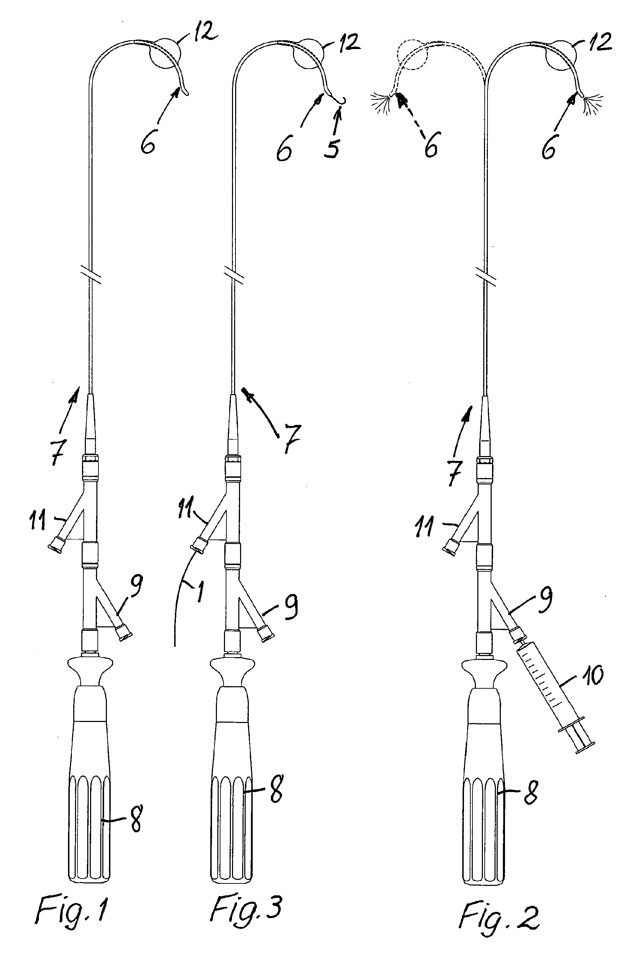

Zu diesem Katheterbausatz gehört ferner ein in den Fig. 1 bis 3 deutlich dargestellter, an seinem distalen Ende 6 lenkbarer, im Ganzen mit 7 bezeichneter Führungskatheter, der im Bereich seines Handgriffes 8 einerseits einen Einlass 9 für ein Medikament aufweist, das beispielsweise mit einer Injektionsvorrichtung 10 eingebracht werden kann, wie es Fig. 2 zeigt, und der einen weiteren Eintritt 11 für den Führungsdraht 1 aufweist, was vor allem in Fig. 3 deutlich ist, wo der Führungsdraht 1 bereits in diesen Führungskatheter 7 eingeschoben ist und mit seinem Ende 5 aus dem distalen Ende 6 des Führungskatheters 7 geringfügig herausragt. Das distale Ende 5 des Führungsdrahtes 1 ist dabei gekrümmt, so dass der Führungsdraht 1 nach dem Erreichen seiner Gebrauchsstellung gemäß den Fig. 4 bis 6 im Gewebe eingehakt werden kann.

Der Führungskatheter 7 hat also einen durchgängigen, an seinem lenkbaren distalen Ende 6 mündenden Kanal oder ein Innenlumen, in welchem der Führungsdraht 1 zumindest bei seiner Verlegung gemäß Fig. 4 angeordnet und in seine Gebrauchslage verschiebbar ist. Somit kann zum Verlegen des Führungsdrahtes 1 die Lenkbarkeit des Führungskatheters 7 ausgenutzt werden, um auf einfache und schnelle Weise den Führungsdraht 1 auch an eine schwierige Stelle, beispielsweise im Koronar-Sinus, bringen zu können. Der Führungsdraht 1 kann zusammen mit dem Führungskatheter 7 - in diesem bereits untergebracht - oder nach dem Einführen des Führungskatheters 7 nachträglich in seine Gebrauchslage gebracht werden.

Der Führungskatheter 7 ist relativ zu dem Führungsdraht 1 auch wieder zurückziehbar, wenn dieser seine Gebrauchslage gemäß Fig. 4 und 5 erreicht hat, und in Fig. 5 ist dieser Vorgang des Zurückziehens des Führungskatheters 7 dargestellt. Gegenüber der in Fig. 4 gezeigten Lage ist das distale Ende 6 des Führungskatheters 7 in Fig. 5 bereits ein Stück weit zurückgezogen und befindet sich kurz vor dem Verlassen des Herzens 2.

Der Kanal oder das Innenlumen des lenkbaren Führungskatheters 7 ist dabei flüssigkeitsdicht, damit die in Fig. 2 angedeutete Möglichkeit, ein Medikament durch diesen Führungskatheter 7 beispielsweise in das Herz 2 einzubringen, bevor der Behandlungskatheter 3 verlegt wird, problemlos möglich ist.

In den Fig. 1 bis 5 erkennt man, dass an dem Führungskatheter 7 im Ausführungsbeispiel nahe dem distalen Ende 6 ein aufweitbarer Ballon 12 angeordnet ist. Damit kann gegebenenfalls beispielsweise in der Anordnung gemäß Fig. 4 der Blutfluss kurzzeitig unterbrochen werden, um die zu behandelnde Stelle beispielsweise mit einer Glasfaseroptik untersuchen zu können.

Im Inneren des Führungskatheters 7 kann ein zu dem Ballon 12 führender eigenständiger Kanal, ein Schlauch, ein Röhrchen oder eine Leitung vorgesehen sein, durch welche das zum Aufweiten des Ballons 12 dienende Medium geleitet werden kann.

Ferner ist in nicht näher dargestellter Weise der lenkbare Führungskatheter 7 mit wenigstens einem exzentrisch zu seiner Längsmitte angeordneten und bewegbaren Zugelement versehen, das zu seinem auslenkbaren biegsamen distalen Ende 6 verläuft und durch eine Zug- und/oder Druckkraft zur Auslenkung des distalen Endes 6 des Führungskatheters 7 dient, das heißt der Führungskatheter 7 kann bezüglich seiner Lenkbarkeit etwa so ausgebildet sein, wie es aus DE 103 37 580 A1 bekannt ist.

Dabei kann das nicht näher dargestellte, im Inneren des Führungskatheters 7 angeordnete Zugelement seinerseits in einem Führungselement oder Röhrchen verlaufen und von diesem sein Ausknicken bei Schubbelastung verhindernd eng umschlossen sein.

Die vorbeschriebene Katheteranordnung, also der Katheterbausatz gemäß den Fig. 1 bis 6 ermöglicht es, einen Führungsdraht 1 zu einer Stelle im Inneren eines Herzens 2 zu verlegen, die mit einem Führungsdraht normalerweise nicht erreichbar ist, indem der Bausatz einen Führungskatheter 7 aufweist, mit welchem oder durch welchen der Führungsdraht 1 verschiebbar ist, wenn dieser Führungskatheter 7 zu der zu erreichenden Stelle hin verlegt ist. Danach kann dann in üblicher Weise nach dem Zurückziehen des Führungskatheters 7 der eigentlich wichtigste Bestandteil des Katheterbausatzes, nämlich der Behandlungskatheter 3 über den Führungsdraht 1 zu der Behandlungsstelle gelangen.

Der Katheterbausatz mit einem Führungsdraht 1 und einem über diesen Führungsdraht 1 zu einem Behandlungsort verschiebbaren Behandlungskatheter 3 weist außerdem noch einen an seinem distalen Ende 6 lenkbaren Führungskatheter 7 auf, der ein Innenlumen hat, um den Führungsdraht 1 in sich aufnehmen zu können. Die zunächst als eine Einheit zusammenwirkende Kombination des lenkbaren Führungskatheters 7 und des Führungsdrahtes 1 ermöglicht die Verlegung dieses Führungsdrahtes 1 auch an schwer zugängliche Stellen. Nach dem Zurückziehen des Führungskatheters 7 kann dann über den verlegten Führungsdraht 1 der Behandlungskatheter 3 in Gebrauchsstellung gebracht werden.

## Patentansprüche

1. Katheterbausatz mit einem Führungsdraht (1) und mit einem auf diesem Führungsdraht (1) zu seinem Bestimmungsort verschiebbaren Behandlungskatheter (3), der einen inneren Kanal oder ein Innenlumen aufweist, womit er in Gebrauchsstellung den Führungsdraht (1) umschließt, **dadurch gekennzeichnet, dass** zu dem Katheterbausatz ein an seinem distalen Ende (6) lenkbarer Führungskatheter (7) gehört, der einen durchgängigen, an seinem lenkbaren distalen Ende (6) mündenden Kanal oder ein Innenlumen aufweist, in welchem der Führungsdraht (1) zumindest bei seiner Verlegung angeordnet und in seine Gebrauchslage verschiebbar ist.

2. Katheterbausatz nach Anspruch 1, **dadurch gekennzeichnet, dass** der Führungskatheter (7) relativ zu dem Führungsdraht (1) verschiebbar und zurückziehbar und/oder entfernbar ist.

3. Katheterbausatz nach Anspruch 1 oder 2, **dadurch gekennzeichnet, dass** der Kanal oder das Innenlumen des lenkbaren Führungskatheters (7) flüssigkeitsdicht ist.

4. Katheterbausatz nach einem der Ansprüche 1 bis 3, **dadurch gekennzeichnet, dass** an dem Führungskatheter (7) am oder nahe dem distalen Ende (6) ein aufweitbarer Ballon (12) angeordnet ist.

5. Katheterbausatz nach einem der Ansprüche 1 bis 4, **dadurch gekennzeichnet, dass** im Inneren des Führungskatheters (7) ein zu dem Ballon (12) führender eigener Kanal, Schlauch, Röhrchen oder Leitung für das zum Aufweiten des Ballons (12) dienenden Medium vorgesehen ist.

6. Katheterbausatz nach einem der Ansprüche 1 bis 5, **dadurch gekennzeichnet, dass** der lenkbare Führungskatheter (7) wenigstens ein exzentrisch zu seiner Längsmitte angeordnetes Zugelement aufweist, das zu seinem auslenkbaren biegsamen distalen Ende (6) verläuft und durch eine Zug- und/oder Druckkraft zur Auslenkung des distalen Endes (6) des Führungskatheters (7) beaufschlagbar ist.

7. Katheterbausatz nach einem der Ansprüche 1 bis 6, **dadurch gekennzeichnet, dass** das Zugelement in einem Führungselement oder Röhrchen verläuft und von diesem sein Ausknicken bei Schubbelastung verhindernd eng umschlossen ist.
